Europäisches Patentamt

(19) European Patent Office

Office européen des brevets

(11) EP 0 531 938 B1

(12) **EUROPEAN PATENT SPECIFICATION**

(45) Date of publication and mention
of the grant of the patent:
**22.01.1997  Bulletin 1997/04**

(51) Int. Cl.[6]: **A61K 9/70**

(21) Application number: **92115344.1**

(22) Date of filing: **08.09.1992**

(54) **Acrylic gel material and gel-based medical preparation for percutaneous absorption employing the same**

Acrylgel und dieses enthaltende Zusammensetzung zur perkutanen Absorption

Gel acrylique, et préparation le contenant pour l'absorption percutanée

(84) Designated Contracting States:
**CH DE ES FR GB IT LI NL**

(30) Priority: **09.09.1991 JP 258442/91**
**09.09.1991 JP 258443/91**
**09.09.1991 JP 258444/91**
**09.09.1991 JP 258445/91**
**20.11.1991 JP 332493/91**

(43) Date of publication of application:
**17.03.1993  Bulletin 1993/11**

(73) Proprietor: **NITTO DENKO CORPORATION**
**Osaka (JP)**

(72) Inventors:
• **Akemi, Hitoshi,**
**c/o Nitto Denko Corporation**
**Ibaraki-shi, Osaka (JP)**
• **Kinoshita, Takashi,**
**c/o Nitto Denko Corporation**
**Ibaraki-shi, Osaka (JP)**
• **Higashio, Kazuhiro,**
**c/o Nitto Denko Corporation**
**Ibaraki-shi, Osaka (JP)**

• **Otsuka, Saburo,**
**c/o Nitto Denko Corporation**
**Ibaraki-shi, Osaka (JP)**
• **Muraoka, Takateru,**
**c/o Nitto Denko Corporation**
**Ibaraki-shi, Osaka (JP)**
• **Kobayashi, Ichiro,**
**c/o Nitto Denko Corporation**
**Ibaraki-shi, Osaka (JP)**
• **Sasaki, Yasuyuki,**
**c/o Nitto Denko Corporation**
**Ibaraki-shi, Osaka (JP)**

(74) Representative: **Grünecker, Kinkeldey,**
**Stockmair & Schwanhäusser**
**Anwaltssozietät**
**Maximilianstrasse 58**
**80538 München (DE)**

(56) References cited:
**EP-A- 0 435 199**              **EP-A- 0 436 203**

Remarks:
The file contains technical information submitted
after the application was filed and not included in
this specification

**Description**

FIELD OF THE INVENTION

The present invention relates to an acrylic ester gel material which is applied to the skin for skin protection or other purposes, and also relates to an acrylic ester gel-based medical preparation (hereinafter often referred to as "gel preparation") for percutaneous absorption which employs the acrylic gel material and is used to continuously administer a drug to a living body through the skin.

BACKGROUND OF THE INVENTION

As covering materials for protecting damaged skin parts or other skin parts and as medical preparations for percutaneous absorption which are used for administering a drug to a living body through the skin, various kinds of covering materials or dressings in tape or sheet form have been proposed which comprise a nonwoven fabric or plastic film having formed on one side thereof a pressure-sensitive adhesive layer. In such covering materials or dressings employing a pressure-sensitive adhesive, the pressure-sensitive adhesive layer is usually required to show proper adhesion to the skin to which the covering material or dressing is applied, in order to prevent the covering material or dressing from falling off the skin. However, as the adhesion of a covering material or dressing to the skin becomes stronger, stripping of the covering material or dressing from the skin gives a stronger physical stimulation to the skin or is more apt to cause a pain to the person and, in some cases, horny layer cells in the skin may be peeled along with the covering material or dressing to cause a damage to the skin. In the case of pressure-sensitive adhesive-based medical preparations, in particular, the above-described problem that stripping of these preparations gives a stimulation to the skin is serious because such a preparation, in most cases, is kept being applied in the same position over a long period of time.

Crosslinked pressure sensitive adhesive layers comprising (meth) acrylic acid alkyl esters have already seen disclosed in EP-A-0 435 199 and EP-A-0 436 203. However, no reference is made in said documents to the importance of a gel content of from 50-80% by weight of the polymer.

Therefore, in developing a covering material or dressing of the above-described kind which is applied to the skin, there has been a desire for a material which has excellent suitability for applying to the skin, i.e., which shows good adhesion or bondability to the skin, is less apt to give a physical stimulation to the skin when stripped therefrom, and does not damage the skin.

Various kinds of dressing-type medical preparations in the form of poultice or tape which employ a salve or pressure-sensitive adhesive have been developed recently as medical preparations for percutaneous absorption, i.e., for administering a drug to a living body through the skin.

As expedients for enabling a dressing-type medical preparation for percutaneous absorption to effectively release a drug therefrom on the skin surface and enabling the released drug to be absorbed by the skin, it has, for example, been proposed to increase the concentration of the drug contained in the preparation and to incorporate a percutaneous absorption-promoting agent along with the drug. In one embodiment of the former expedient, a drug is incorporated at a supersaturated concentration, based on the idea that a drug contained in a dissolved state is released to the skin surface. However, it is not easy to maintain the supersaturated state of the drug in a produced medical preparation until use, and there are cases that crystals of the drug separate out during storage and this results in poor percutaneous absorption.

On the other hand, the latter expedient is defective in that the absorption-promoting agent may stimulate the skin to which the dressing-type medical preparation is applied or may adversely affect the tackiness properties of the pressure-sensitive adhesive or the stability of the drug, although the percutaneous absorption of the drug is improved due to the presence of the absorption-promoting agent in the salve or pressure-sensitive adhesive.

Furthermore, it may be thought that the percutaneous absorption of a drug can be promoted by increasing the drug content by either employing a pressure-sensitive adhesive or the like in which the drug can dissolve in a large amount or incorporating an agent which increases solubility of the drug (solubilizing agent). However, there are cases that the drug becomes less apt to be released because distribution of the drug to the pressure-sensitive adhesive is generally enhanced.

SUMMARY OF THE INVENTION

Under these circumstances, the present inventors have conducted intensive studies to overcome the above-described problems. As a result, it has been found that a good balance between adhesion to the skin and skin-unstimulating properties can be attained when, in preparing a pressure-sensitive adhesive using an acrylic ester-based polymer, as a major component, obtained mainly form an alkyl (meth)acrylate, a liquid ingredient compatible with the polymer is incorporated in an amount larger than normal amounts, and the polymer is crosslinked to allow it to gel to a degree such that the resulting pressure-sensitive adhesive layer has a gel content in a specific range, thereby to remedy the decreased cohesive force due to the incorporation of the liquid ingredient and, at the same time, to enable the pressure-sensitive adhesive to show soft adhesion to the skin and to ease or disperse a stress to be imposed on the skin when the adhesive layer is stripped from the skin. The present invention has been completed based on the above.

Accordingly, an object of the present invention is to provide an acrylic ester gel material as defined in claim 1.

Another object of the present invention is to provide an acrylic ester gel-based medical preparation for percutaneous absorption as defined in claim 11.

## DETAILED DESCRIPTION OF THE INVENTION

The substrate for use in the acrylic gel material and the gel preparation for percutaneous absorption according to the present invention is not particularly limited. Preferably, however, the substrate is not a material through which the liquid component or drug contained in the pressure-sensitive adhesive layer can pass and which thus allows part of these ingredients to be lost, resulting in reduced ingredient contents. That is, it is preferred that the substrate is made of a material impermeable to these ingredients. Examples of the substrate include single-layer films such as films of polymers, e.g., a polyester, nylon, saran, polyethylene, polypropylene, ethylene-vinyl acetate copolymer, poly(vinyl chloride), ethylene-ethyl acrylate copolymer, polytetrafluoroethylene, and Surlyn, and metal foils and laminates of these films. From the standpoint of attaining improved adhesion (anchoring effect) between the substrate and a pressure-sensitive adhesive layer to be described later, it is preferred to employ, among those examples, a laminate of a non-porous sheet and a porous sheet each made of any of the above materials and to form a pressure-sensitive adhesive layer on the porous sheet side.

As the porous sheet, any porous sheet may be used without particular limitation so long as a pressure-sensitive adhesive layer to be formed thereon can show improved anchoring effect. Examples of the porous sheet include papers, woven fabrics, nonwoven fabrics, and mechanically perforated sheets. Particularly preferred of these are papers, woven fabrics, and nonwoven fabrics. The thickness of the porous sheet generally is from 10 to 500 $\mu$m from the standpoints of attaining improved anchoring effect and imparting good flexibility to the whole gel material or gel preparation. When the gel material or gel preparation is relatively thin as in the case of dressing type or pressure-sensitive tape type, the thickness of the porous sheet generally is from 10 to 200 $\mu$m.

In the case of using a woven or nonwoven fabric as the porous sheet, it is preferable that the fabric have a weight per unit area of the woven or nonwoven fabric of from 5 to 30 g/m$^2$, preferably from 8 to 20 g/m$^2$, from the standpoint of attaining improved anchoring effect.

The pressure-sensitive adhesive layer formed on one side of the substrate in the present invention is a crosslinked pressure-sensitive adhesive layer which contains an acrylic ester-based polymer and a liquid component compatible with the polymer and which has both appropriate adhesion to the skin and appropriate cohesive force. One measure of the adhesion properties of the pressure-sensitive adhesive layer is adhesion strength to a Bakelite plate, which is generally from about 20 to 200 g/12mm-width, preferably from about 30 to 180 g/12mm-width (the measurement method will be described after).

Another measure of the adhesion properties of the pressure-sensitive adhesive layer is adhesion strength to the same pressure-sensitive adhesive layer, which is determined by a method in which two gel material samples or gel preparation samples having the same pressure-sensitive adhesive layer are superposed on each other with the pressure-sensitive adhesive layers facing each other, subsequently the superposed samples are bonded to each other by applying a load thereto, and the adhesion strength between the two pressure-sensitive adhesive layers is measured by a 180° constant-rate peel strength test. It is desirable that this adhesion strength is from 20 to 180 g/12mm-width, preferably from 30 to 165 g/12mm-width, more preferably from 40 to 150 g/12mm-width.

It is also preferable that the pressure-sensitive adhesive layer is made of an elastic material which, even when a shearing stress is imposed thereon, suffers neither cohesive failure or interfacial failure, and that the shear-deformation-shift completion distance for the pressure-sensitive adhesive layer on which a shearing stress is being imposed is from 0.5 to 6 times the thickness of the pressure-sensitive adhesive layer.

Moreover, it is preferable that the adhesion strength of the pressure-sensitive adhesive layer to the skin is such that the interfacial adhesion strength between the pressure-sensitive adhesive layer and horny layer cells is substantially lower than the bonding strength among the horny layer cells.

The acrylic ester-based polymer used in the present invention is a polymer obtained mainly from a (meth)acrylic acid alkyl ester, which is one of major raw materials for pressure-sensitive adhesives. The acrylic ester-based polymer is a major basic material constituting the pressure-sensitive adhesive layer along with the liquid component that will be described later. This polymer in the pressure-sensitive adhesive layer maintains a homogeneously mixed state with the liquid component and enables the pressure-sensitive adhesive layer to exhibit good adhesion to the skin and good retention of shape. Use of a rubber, e.g., natural rubber or a synthetic rubber, or a silicone polymer is not preferred because these are not sufficiently compatible with the liquid component used in the present invention or have extremely poor drug-dissolving or drug-releasing properties. In addition, rubbers and silicone polymers are also defective in that these are difficult to crosslink in a reproducible manner because it is difficult to adjust the amount of functional groups and the like which participate in crosslinking reactions, as compared with the case of acrylic ester-based polymers. For these reasons, rubbers and silicones are unsuitable for use in the present invention.

The acrylic ester-based polymer used in the present invention preferably is a polymer obtained using an alkyl (meth)acrylate having 4 or more, preferably from 4 to 15, carbon atoms in the alkyl moiety. It is particularly desirable to use a copolymer obtained using such the alkyl (meth)acrylate as the main monomer, from the standpoint of easiness in crosslinking treatment.

More preferably, the acrylic ester-based polymer is a polymer obtained by copolymerizing from 60 to 98% by weight of the alkyl (meth)acrylate having from 4 to 15 carbon atoms in the alkyl moiety and from 2 to 40% by weight of a monomer copolymerizable with the alkyl (meth)acrylate.

The alkyl (meth)acrylate may be one in which the alkyl group is either straight-chain or branched. Specific examples of the alkyl (meth)acrylate include butyl, pentyl, hexyl, heptyl, octyl, nonyl, decyl, undecyl, dodecyl, and tridecyl (meth)acrylates. These may be used alone or in combination of two or more thereof.

Examples of the monomers that can be copolymerized with the alkyl (meth)acrylate include carboxyl group-containing monomers such as (meth)acrylic acid, itaconic acid, maleic acid, and maleic anhydride; sulfoxyl group-containing monomers such as styrenesulfonic acid, allylsulfonic acid, sulfopropyl (meth)acrylate, (meth)acryloyloxynaphthalenesulfonic acid, and acrylamidomethylpropanesulfonic acid; hydroxyl group-containing monomers such as hydroxyethyl (meth)acrylate and hydroxypropyl (meth)acrylate; amide group-containing monomers such as (meth)acrylamide, dimethyl(meth)acrylamide, N-butylacrylamide, N-methylol(meth)acrylamide, and N-methylolpropane(meth)acrylamide; alkylaminoalkyl group-containing monomers such as aminoethyl (meth)acrylate, dimethylaminoethyl (meth)acrylate, and t-butylaminoethyl (meth)acrylate; alkoxyalkyl (meth)acrylates such as methoxyethyl (meth)acrylate and ethoxyethyl (meth)acrylate; (meth)acrylic acid esters containing an alkoxy group (or a pendant group having an ether bond) such as tetrahydrofurfuryl (meth)acrylate, methoxyethylene glycol (meth)acrylate, methoxydiethylene glycol (meth)acrylate, methoxypolyethylene glycol (meth)acrylate, and methoxypolypropylene glycol (meth)acrylate; and vinyl monomers such as (meth)acrylonitrile, vinyl acetate, vinyl propionate, N-vinyl-2-pyrrolidone, methylvinylpyrrolidone, vinylpyridine, vinylpiperidone, vinylpyrimidine, vinylpiperadine, vinylpyrazine, vinylpyrrole, vinylimidazole, vinylcaprolactam, vinyloxazole, and vinylmorpholine. These may be used alone or in combination of two or more thereof, in copolymerization with the alkyl (meth)acrylate. These co-monomers that can be copolymerized with the alkyl (meth)acrylate may be used for the purpose of adjusting the cohesive force of the pressure-sensitive adhesive layer to be formed or improving the drug-dissolving power of the pressure-sensitive adhesive layer. The amount of such co-monomer(s) to be copolymerized with the alkyl (meth)acrylate may be arbitrarily determined according to the use of the gel material or gel preparation to be produced, in the range of from 2 to 40% by weight, preferably from 3 to 35% by weight, based on the total amount of all the monomers.

Of such various acrylic ester-based polymers, copolymers obtained by copolymerizing an alkyl (meth)acrylate and at least one of the above-described carboxyl group-containing monomers and hydroxyl group-containing monomers, as essential components, and if required and necessary, one or more of the other monomers described above are advantageously used in the present invention from the standpoint of control of the amount of crosslinking sites or control of tackiness properties.

The organic liquid component used in the present invention has the property of being compatible with the above-described acrylic ester-based polymer. This liquid component plasticizes the polymer to impart softness to the pressure-sensitive adhesive and thereby serves, when the pressure-sensitive adhesive layer is stripped from the skin, to prevent horny layer cells being peeled from the skin. Accordingly, the organic liquid component is an important ingredient because it functions to diminish the pain or stimulation which is caused to the skin by stripping of the pressure-sensitive adhesive layer due to its adhesion to the skin.

As the organic liquid component, any organic liquid may be used so long as it has a plasticizing effect to the pressure-sensitive adhesive. The amount of the organic liquid component contained in the pressure-sensitive adhesive layer is large as compared with those for conventional gel materials or gel preparations.

Examples of the organic liquid component include glycols such as ethylene glycol, diethylene glycol, triethylene glycol, propylene glycol, polyethylene glycol, and polypropylene glycol; oils and fats such as olive oil, castor oil, squalene, and lanolin; organic solvents such as oleic acid, ethyl acetate, ethyl alcohol, 1,3-butanediol, dimethyldecyl sulfoxide, methyl octyl sulfoxide, dimethyl sulfoxide, dimethylformamide, dimethylacetamide, dimethyllaurylamide, dodecylpyrrolidone, and isosorbitol; liquid surfactants; plasticizers such as diisopropyl adipate, phthalic acid esters, and diethyl sebacate; hydrocarbons such as liquid paraffin; and esters such as glycerin esters, isopropyl myristate, isotridecyl myristate, ethyl laurate, N-methylpyrrolidone, ethyl oleate, diisopropyl adipate, isopropyl palmitate, and octyl palmitate. These may be contained alone or in combination of two or more thereof.

The amount of the liquid component contained in the pressure-sensitive adhesive layer is generally from 25 to 200 parts by weight, preferably from 40 to 160 parts by weight, more preferably from 100 to 160 parts by weight, per 100 parts by weight of the acrylic ester-based polymer. When a liquid component is contained in the crosslinked pressure-sensitive adhesive layer in a large amount as in the case of the present invention, the adhesion strength (interfacial adhesion strength) of the pressure-sensitive adhesive layer to the skin becomes lower than the bonding strength among

EP 0 531 938 B1

horny layer cells, so that stripping of the pressure-sensitive adhesive layer from the skin does not result in peeling of horny layer cells. A measure of such an interfacial adhesion strength (adhesion strength to the skin) is that the amount of horny layer cells which have peeled off the skin upon stripping of the pressure-sensitive adhesive layer from the skin and are adhered to the pressure-sensitive adhesive layer is about 1.0 or less in terms of the absorbance of an extract obtained using the stripped pressure-sensitive adhesive layer. The absorbance-measuring method in the present invention will be described after.

In preparing the acrylic gel material according to the present invention, a pressure-sensitive adhesive comprising the above-described components in respective amounts as specified above is crosslinked by a suitable crosslinking means thereby to form a pressure-sensitive adhesive layer having a gel content of from 50 to 80% by weight. Due to this crosslinking, the liquid component contained in the pressure-sensitive adhesive layer is prevented from oozing therefrom and cohesive force is imparted to the pressure-sensitive adhesive layer. The crosslinking may be accomplished by a physical treatment such as irradiation with ultraviolet rays or electron beams or by a chemical treatment using a crosslinking agent such as a polyisocyanate compound, organic peroxide, organic metal salt, metal alcoholate, metal chelate compound, or polyfunctional compound. Of these crosslinking means, irradiation treatment and treatment with an organic peroxide may cause a decomposition reaction of a drug, if any, according to the kind of the drug. Use of a highly reactive isocyanate compound or a metal salt or organic metal salt for use in ordinary crosslinking reactions is disadvantageous in that the pressure-sensitive adhesive solution may undergo a viscosity increase after the addition of the crosslinking agent to show poor workability. Further, although it may be possible to use a method in which a polyfunctional monomer such as a diacrylate is copolymerized beforehand with the acrylic ester-based polymer, the pressure-sensitive adhesive solution obtained by this method may also increase in viscosity.

Therefore, of the above-described crosslinking agents, trifunctional isocyanates and metal alcoholate or chelate compounds in which the metals are titanium or aluminum are preferred in the present invention from the standpoints of reactivity and handling properties. These preferred crosslinking agents do not cause pressure-sensitive adhesive solutions to undergo a viscosity increase until coating and drying, thus enabling the coating solutions to show extremely good workability. In the case of using such a crosslinking agent, it is important that during preparation of a pressure-sensitive adhesive, the crosslinking agent should be used in an amount of from about 0.05 to 3.0 parts by weight per 100 parts by weight of the acrylic ester-based polymer, so as to result in a gel content in the above-specified range. If the pressure-sensitive adhesive layer formed has a gel content below 50% by weight, sufficient cohesive force cannot be imparted to the pressure-sensitive adhesive layer and, as a result, when the pressure-sensitive adhesive layer is stripped from the skin, part of the pressure-sensitive adhesive remains on the skin due to the cohesive failure of the pressure-sensitive adhesive layer or the skin receives a considerable stimulation. On the other hand, if the gel content exceeds 80% by weight, the pressure-sensitive adhesive layer cannot have sufficient adhesion strength to the skin, although its cohesive force is high.

According to the present invention, by containing a drug in the thus-formed pressure-sensitive adhesive layer, a gel-based medical preparation is obtained.

In the gel preparation, the pressure-sensitive adhesive layer containing a drug serves to release the drug when the gel preparation is applied to the skin. The pressure-sensitive adhesive which is a main constituent of the pressure-sensitive adhesive layer is required to have an appropriate drug-dissolving power so as to stably release the drug contained therein. For use in the gel preparation of the present invention, a pressure-sensitive adhesive in which the solubility of the drug used is 4% by weight or more is generally selected. If the solubility of a drug in a pressure-sensitive adhesive is below 4% by weight, the amount of the drug that can be retained in a dissolved state by the pressure-sensitive adhesive is limited, so that administration of the drug in an amount necessary for cure is difficult and the effect of the present invention cannot be produced sufficiently.

Along with the pressure-sensitive adhesive, an organic liquid component compatible with the pressure-sensitive adhesive is contained in the pressure-sensitive adhesive layer of the gel preparation. This organic liquid component functions to decrease the solubility of the drug contained in the pressure-sensitive adhesive layer and thereby to control release of the drug. As this organic liquid component, a material in which the solubility of the drug used is 3% by weight or less is generally selected. If an organic liquid component in which the solubility of the drug is higher than 3% by weight is used, the effect of the present invention cannot be produced sufficiently because such an organic liquid component does not sufficiently perform its desired function due to the relationship between the drug solubility in the organic liquid component and that in the pressure-sensitive adhesive.

The drug contained in the pressure-sensitive adhesive layer can be arbitrarily selected from various percutaneously absorbable drugs according to the medical treatment for which the gel preparation is to be used. Examples of the drug include corticosteroids, analgesic anti-inflammatory drugs, hypnotic sedatives, tranquilizers, antihypertensive drugs, hypotensive diuretics, antibiotics, anesthetics, antibacterial drugs, antifungal drugs, Vitamines, vasodilators for coronary artery, antihistaminics, antitussives, sex hormones, thymoleptics, ameliorants of cerebral circulation, antiemetic drugs, antitumor drugs, and vital medicines. If required and necessary, two or more of these drugs may be used in combination. Of the above-described drugs, fat-soluble drugs (solubility: 0.4 g or less in 100 ml water at room temperature) are preferably used from the standpoints of dispersibility into the pressure-sensitive adhesive layer and percutaneous absorption.

The amount of such a drug contained in the pressure-sensitive adhesive layer can be suitably determined according to the kind of the drug and the purpose of administration. Normally, however, pressure-sensitive adhesive layer contains a drug in an amount of from about 1 to 40% by weight, preferably from about 3 to 30% by weight, based on the weight of the pressure-sensitive adhesive layer. If the drug content in the pressure-sensitive adhesive layer is below 1% by weight, there are cases that release of the drug in an amount useful for the intended medical treatment cannot be expected. Drug contents exceeding 40% by weight not only are undesirable in that the treating effect does not increase any more even with increasing drug content, but are economically disadvantageous.

The acrylic gel material and gel preparation for percutaneous absorption according to the present invention, which respectively have the constructions described above, are characterized in that the pressure-sensitive adhesive layer contains a large amount of an organic liquid component compatible with the acrylic ester-based polymer and that due to this, the pressure-sensitive adhesive layer can be soft while retaining sufficient cohesive force and, hence, attains diminished skin-stimulating properties. Therefore, the medical preparation of the present invention is less apt to cause a pain or skin stimulation, which may result due to the adhesion of pressure-sensitive adhesive layer, when the preparation is stripped from the skin to which it has been applied, and the medical preparation can have a good balance between appropriate adhesion to the skin and skin-unstimulating properties. Furthermore, the medical preparation for percutaneous absorption in which a drug has been contained in the pressure-sensitive adhesive layer can release the drug on the skin surface at an appropriate rate and is, therefore, useful in the treatment or prevention of various diseases in which a drug is administered to a living body through the skin by percutaneous absorption.

The present invention will be explained below in more detail with reference to the following examples, but the invention is not construed as being limited thereto. In these Examples and Comparative Examples, all parts and percents are by weight.

EXAMPLE 1

43 Parts of an acrylic ester-based polymer obtained by copolymerizing 95 parts of 2-ethylhexyl acrylate and 5 parts of acrylic acid in ethyl acetate in an inert gas atmosphere was mixed with 40 parts of isopropyl myristate and 0.06 part of a trifunctional isocyanate (Coronate HL, manufactured by Nippon Polyurethane Industry Co., Ltd., Japan) in ethyl acetate, thereby preparing a pressure-sensitive adhesive solution.

The solution obtained was coated on a polyester separator at a dry thickness of 60 $\mu$m, and the coating was dried to form a crosslinked pressure-sensitive adhesive layer. This pressure-sensitive adhesive layer was transferred to a substrate film which was a laminate of a polyester nonwoven fabric (12 g/m$^2$) and a polyester film (2 $\mu$m thick), on the nonwoven fabric side. Thus, an acrylic gel material according to the present invention was obtained.

EXAMPLE 2

The same procedures as in Example 1 were conducted except that the pressure-sensitive adhesive solution prepared in Example 1 was coated after 17 parts of isosorbide dinitrate was added thereto. Thus, an acrylic gel preparation according to the present invention was obtained in which the pressure-sensitive adhesive layer contained a liquid component and had been crosslinked.

COMPARATIVE EXAMPLE 1

An acrylic gel material was obtained in the same manner as in Example 1 except that the pressure-sensitive adhesive was not crosslinked (i.e., trifunctional isocyanate was not added). This gel material showed considerable cohesive failure, so that its adhesive strength and other properties were unable to be evaluated in the tests described after.

EXAMPLE 3

An acrylic gel preparation according to the present invention was obtained in the same manner as in Example 2 except that 0.09 part of (ethyl acetoacetate)aluminum diisopropylate was used as a crosslinking agent in place of 0.06 part of the trifunctional isocyanate.

COMPARATIVE EXAMPLE 2

The same procedures as in Example 1 were conducted except that isopropyl myristate and the isocyanate were not added to the pressure-sensitive adhesive solution and that the adhesive solution was mixed with 17 parts of isosorbide dinitrate per 83 parts of the solid component of the solution. Thus, an acrylic gel preparation was obtained in which the pressure-sensitive adhesive layer did not contain liquid component and had not been crosslinked.

COMPARATIVE EXAMPLE 3

An acrylic gel preparation in which the pressure-sensitive adhesive layer did not contain liquid component was obtained in the same manner as in Comparative Example 2 except that 0.12 part of the trifunctional isocyanate was further added to the pressure-sensitive adhesive solution to crosslink the pressure-sensitive adhesive layer.

COMPARATIVE EXAMPLE 4

An acrylic gel preparation was obtained in the same manner as in Example 3 except that the amount of (ethyl acetoacetate)aluminum diisopropylate added was changed to 0.02 part.

EXAMPLE 4

60 Parts of an acrylic ester-based polymer obtained by copolymerizing 75 parts of 2-ethylhexyl acrylate, 22 parts of N-vinyl-2-pyrrolidone, and 3 parts of acrylic acid in ethyl acetate in an inert gas atmosphere was mixed with 40 parts of octyl palmitate and 0.12 part of a trifunctional isocyanate (Coronate HL, manufactured by Nippon Polyurethane Industry Co,. Ltd.) in ethyl acetate, thereby preparing a pressure-sensitive adhesive solution.
The solution obtained was coated on a separator and dried in the same manner as in Example 1 to form a crosslinked pressure-sensitive adhesive layer. This pressure-sensitive adhesive layer was transferred to a substrate film which was a laminate of a polyester nonwoven fabric (12 g/m$^2$) and a polyester film (2 $\mu$m thick), on the nonwoven fabric side. Thus, an acrylic gel material according to the present invention was obtained.

EXAMPLE 5

The same procedures as in Example 4 were conducted except that a pressure-sensitive adhesive solution was prepared by mixing 57.5 parts of the same acrylic ester-based polymer as prepared in Example 4, 40 parts of octyl palmitate, 2.5 parts of estradiol, and 0.115 part of a trifunctional isocyanate (Coronate HL, manufactured by Nippon Polyurethane Industry Co., Ltd.) in ethyl acetate. Thus, an acrylic gel preparation according to the present invention was obtained

COMPARATIVE EXAMPLE 5

An acrylic gel material was obtained in the same manner as in Example 4 except that the pressure-sensitive adhesive was not crosslinked (i.e., the trifunctional isocyanate was not added). This gel material showed considerable cohesive failure, so that its adhesive strength and other properties were unable to be evaluated in the tests described after.

EXAMPLE 6

An acrylic gel preparation according to the present invention was obtained in the same manner as in Example 5 except that 0.0575 part of (ethyl acetoacetate)aluminum diisopropylate was used as a crosslinking agent in place of 0.115 part of the trifunctional isocyanate.

COMPARATIVE EXAMPLE 6

The same procedures as in Example 4 were conducted except that octyl palmitate and the isocyanate were not added to the pressure-sensitive adhesive solution and that the adhesive solution was mixed with 2.5 parts of estradiol per 97.5 parts of the solid component of the solution. Thus, an acrylic gel preparation was obtained in which the pressure-sensitive adhesive layer did not contain liquid component and had not been crosslinked.

COMPARATIVE EXAMPLE 7

An acrylic gel preparation in which the pressure-sensitive adhesive layer did not contain liquid component was obtained in the same manner as in Comparative Example 6 except that 0.195 part of the trifunctional isocyanate was further added to the pressure-sensitive adhesive solution to crosslink the pressure-sensitive adhesive layer.

COMPARATIVE EXAMPLE 8

An acrylic gel preparation was obtained in the same manner as in Example 6 except that the amount of (ethyl acetoacetate)aluminum diisopropylate added was changed to 0.253 part.

EXAMPLE 7

60 Parts of an acrylic ester-based polymer obtained by copolymerizing 90 parts of 2-ethylhexyl acrylate and 10 parts of 2-hydroxyethyl acrylate in ethyl acetate in an inert gas atmosphere was mixed with 40 parts of 1,3-butanediol and 0.10 part of a trifunctional isocyanate (Coronate HL, manufactured by Nippon Polyurethane Industry Co., Ltd.) in toluene, thereby preparing a pressure-sensitive adhesive solution.

The solution obtained was coated on a separator at a dry thickness of 60 $\mu$m and dried in the same manner as in Example 1 to form a crosslinked pressure-sensitive adhesive layer. This pressure-sensitive adhesive layer was transferred to a substrate film which was a laminate of a polyester nonwoven fabric (12 g/m$^2$) and a polyester film (2 $\mu$m thick), on the nonwoven fabric side. Thus, an acrylic gel material according to the present invention was obtained.

COMPARATIVE EXAMPLE 9

An acrylic gel material was obtained in the same manner as in Example 7 except that the pressure-sensitive adhesive was not crosslinked (i.e., the trifunctional isocyanate was not added). This gel material showed considerable cohesive failure, so that its adhesive strength and other properties were unable to be evaluated in the tests described after.

COMPARATIVE EXAMPLE 10

The same procedures as in Example 7 were conducted except that 1,3-butanediol and the isocyanate were not added to the pressure-sensitive adhesive solution and that the adhesive solution was mixed with 10 parts of ketoprofen per 90 parts of the solid component of the solution. Thus, an acrylic gel preparation was obtained in which the pressure-sensitive adhesive layer did not contain liquid component and had not been crosslinked.

COMPARATIVE EXAMPLE 11

An acrylic gel preparation in which the pressure-sensitive adhesive layer did not contain liquid component was obtained in the same manner as in Comparative Example 10 except that 0.144 part of the trifunctional isocyanate was further added as a crosslinking agent to the pressure-sensitive adhesive solution.

EXAMPLE 8

The same procedures as in Example 7 were conducted except that a pressure-sensitive adhesive solution was prepared by mixing 50 parts of the same acrylic ester-based polymer as prepared in Example 7, 40 parts of 1,3-butanediol, 10 parts of ketoprofen, and 0.08 part of the trifunctional isocyanate in toluene. Thus, an acrylic gel preparation according to the present invention was obtained.

COMPARATIVE EXAMPLE 12

The same procedures as in Example 1 were conducted except that isopropyl myristate and the isocyanate were not added to the pressure-sensitive adhesive solution. Thus, an acrylic polymer-based plaster was obtained in which the pressure-sensitive adhesive layer did not contain liquid component and had not been crosslinked.

Each of the samples obtained in the Examples and Comparative Examples given above was stored at 70°C for 2 days and then subjected to the following tests. The results obtained are shown in Table 1.

[Gel Content]

Each sample was cut into 40 cm$^2$ and the weight ($W_1$) of the pressure-sensitive adhesive layer was measured. The sample was then immersed in ethyl acetate for 3 days to extract any solvent-soluble component. Thereafter, the sample was taken out from the ethyl acetate and dried. The weight ($W_2$) of the resulting pressure-sensitive adhesive layer was then measured, and the gel content of the pressure-sensitive adhesive layer was calculated using the following equation:

$$\text{Gel content} = (W_2 \times 100)/(W_1 \times A/B)$$

wherein A = weight of (pressure-sensitive adhesive + crosslinking agent) and B = weight of (pressure-sensitive adhesive + liquid component + crosslinking agent), provided that when the pressure-sensitive adhesive layer contains a drug, the weight of the drug is added to B.

8

[Adhesion Strength Test]

Each sample which had been cut into a 12 mm-wide strip was placed on a Bakelite plate and bonded thereto by moving a 850 g roller forward and backward once on the strip. The strip was then peeled from the plate in the 180° direction at a rate of 300 mm/min, and the peel force required for the peeling was measured.

[Adhesion to Skin]

Each sample was applied to volunteers in their upper arm side parts, and 24 hours later, the adhesion state of each applied sample was visually evaluated. The evaluation was made in 5 grades, with the worst being rated as 1 and the best as 5 (average of 6 samples for each example).

[Skin-Stimulating Property]

After the above test for examining adhesion to the skin, each sample was stripped from the skin and the physical stimulation (pain) which the stripping gave to the skin was evaluated. The evaluation was made in 5 grades, with samples that caused the most slight pain being rated as 1 and samples that caused the most severe pain as 5 (average of 6 samples for each example).

[Adhesive Remaining]

After the above test for examining adhesion to the skin, each sample was stripped from the skin and whether part of the pressure-sensitive adhesive layer remained on the skin due to cohesive failure was visually evaluated.

TABLE 1

| | Gel content (%) | Adhesion strength (g) | Adhesion to skin | Skin-stimulating property | Adhesive remaining |
|---|---|---|---|---|---|
| Example 1 | 59.3 | 75 | 4.8 | 1.2 | None |
| Example 2 | 64.9 | 60 | 5.0 | 1.0 | None |
| Example 3 | 64.2 | 63 | 4.8 | 1.3 | None |
| Example 4 | 58.2 | 123 | 4.7 | 1.5 | None |
| Example 5 | 58.9 | 116 | 5.0 | 1.2 | None |
| Example 6 | 65.1 | 96 | 4.8 | 1.2 | None |
| Example 7 | 74.2 | 48 | 4.7 | 1.3 | None |
| Example 8 | 74.2 | 53 | 4.8 | 1.2 | None |
| Comparative Example 1 | 0 | Measurement impossible | - | - | - |
| Comparative Example 2 | 0 | 335 | 4.8 | 3.7 | None |
| Comparative Example 3 | 66.1 | 288 | 4.8 | 3.3 | None |
| Comparative Example 4 | 29.6 | 212 | 4.2 | 4.3 | Present |
| Comparative Example 5 | 0 | Measurement impossible | - | - | - |
| Comparative Example 6 | 0 | 451 | 4.5 | 4.7 | Slightly present |
| Comparative Example 7 | 60.1 | 432 | 4.3 | 4.2 | None |
| Comparative Example 8 | 88.3 | 23 | 1.2 | 1.2 | None |
| Comparative Example 9 | 0 | Measurement impossible | - | - | - |
| Comparative Example 10 | 0 | 531 | 4.2 | 4.5 | Slightly present |
| Comparative Example 11 | 63.8 | 507 | 4.3 | 4.7 | None |
| Comparative Example 12 | 0 | 414 | 4.0 | 5.0 | None |

EXAMPLE 9

In an inert gas atmosphere, 72 parts of 2-ethylhexyl acrylate, 25 parts of N-vinyl-2-pyrrolidone, and 3 parts of acrylic acid were copolymerized in ethyl acetate, thereby preparing a solution of an acrylic pressure-sensitive adhesive.

To the pressure-sensitive adhesive solution prepared above were added 3 parts of estradiol and 50 parts of isopropyl myristate per 47 parts of the solid component of the solution. Thereto was further added 0.3 part of (ethyl acetoacetate)aluminum diisopropylate crosslinking agent (as acetylacetone solution) per 99.7 parts of the solid component of the pressure-sensitive adhesive solution. The resulting composition was coated on a polyester separator at a dry thickness of 60 μm, and the coating was dried to form a pressure-sensitive adhesive layer. This pressure-sensitive adhesive layer was transferred to a laminate of a polyester nonwoven fabric (12 g/m$^2$) and a polyester film (2 $\mu$m thick), on the nonwoven fabric side. The resulting structure was aged at 70°C to obtain a medical preparation for percutaneous absorption according to the present invention.

COMPARATIVE EXAMPLE 13

A medical preparation for percutaneous absorption was obtained in the same manner as in Example 9 except that a coating composition for forming a pressure-sensitive adhesive layer was prepared by adding 5 parts of estradiol to the pressure-sensitive adhesive solution per 95 parts of the solid component of the solution, without adding isotridecyl myristate.

COMPARATIVE EXAMPLE 14

A medical preparation for percutaneous absorption was obtained in the same manner as in Example 9 except that a coating composition for forming a pressure-sensitive adhesive layer was prepared by adding 4 parts of estradiol and 10 parts of isopropyl myristate to the pressure-sensitive adhesive solution per 86 parts of the solid component of the solution, and that the pressure-sensitive adhesive was crosslinked by adding 0.3 part of (ethyl acetoacetate)aluminum diisopropylate per 99.7 parts of the solid component of the pressure-sensitive adhesive solution.

COMPARATIVE EXAMPLE 15

In an inert gas atmosphere, 95 parts of 2-ethylhexyl acrylate and 5 parts of acrylic acid were copolymerized in ethyl acetate, thereby preparing a solution of an acrylic pressure-sensitive adhesive.

To this solution were added 3 parts of estradiol and 27 parts of isopropyl myristate per 60 parts of the solid component of the solution. A medical preparation for percutaneous absorption was then obtained in the same manner as in Example 9 except that the thus-prepared coating composition was used to form a pressure-sensitive adhesive layer.

The solubility of estradiol in the above-prepared pressure-sensitive adhesive was 1.7%, while that in isopropyl myristate was 0.20%. When the medical preparation obtained above was stored at 40°C for 2 weeks under high humidity conditions of 75%, crystals of estradiol in the form of fine particles separated out in a large number. However, this preparation in such state was subjected, as it was, to the following test.

Each of the medical preparations for percutaneous absorption, obtained in Examples 9 and 10 and Comparative Examples 13 to 15, was cut into 4 cm x 5 cm and then applied to a rabbit on its back part which had been depilated. Forty-eight hours later, each sample was stripped from the rabbit and the content of the drug remaining in the resulting preparation was measured. From a difference in drug content between the sample before application and that after application, the amount of the drug which had been transferred to the skin was calculated. The number of samples tested for each example was 3. The results obtained are shown in Table 2.

Further, the medical preparations were also evaluated for skin-stimulating properties, and the order of the degree of stimulation to the skin was as follows.

Comparative Example 14 $\geq$ Comparative Example 13 > Comparative Example 15 $\geq$ Example 9

Table 2

| | Amount of transferred estradiol ($\mu$g) |
|---|---|
| Example 9 | 1,630 $\pm$ 210 |
| Comparative Example 13 | 90 $\pm$ 70 |
| Comparative Example 14 | 180 $\pm$ 110 |
| Comparative Example 15 | 120 $\pm$ 70 |

EXAMPLE 10

To the pressure-sensitive adhesive solution prepared in Comparative Example 15 were added 20 parts of isosorbide dinitrate and 40 parts of isopropyl myristate per 40 parts of the solid component of the solution. Thereto was further added 0.15 part of a trifunctional isocyanate (crosslinking agent) per 99.85 parts of the solid component of the pressure-sensitive adhesive solution. In the same manner as in Example 9, the thus-obtained composition was coated on a separator at a dry thickness of 60 $\mu$m and dried, and the resulting pressure-sensitive adhesive layer was transferred to a laminate of a polyester nonwoven fabric (12 g/m$^2$) and a polyester film (2 $\mu$m thick), on the nonwoven fabric side and then aged at 70°C. Thus, a medical preparation for percutaneous absorption according to the present invention was obtained.

The solubility of isosorbide dinitrate in the above-prepared pressure-sensitive adhesive was 7%, while that in isopropyl myristate was 2.1%.

EXAMPLE 11

A medical preparation for percutaneous absorption according to the present invention was obtained in the same manner as in Example 10 except that a coating composition for forming a pressure-sensitive adhesive layer was prepared by adding, to the pressure-sensitive adhesive solution prepared in Comparative Example 15, 7 parts of ketoprofen and 43 parts of isotridecyl myristate per 50 parts of the solid component of the solution and further adding 0.15 part of a trifunctional isocyanate (cross-linking agent) per 99.85 parts of the solid component of the pressure-sensitive adhesive solution.

The solubility of ketoprofen in the above-prepared pressure-sensitive adhesive was 8.3%, while that in isopropyl myristate was 2.2%.

COMPARATIVE EXAMPLE 16

A medical preparation for percutaneous absorption was obtained in the same manner as in Comparative Example 15 except that a coating composition for forming a pressure-sensitive adhesive layer was prepared by adding 20 parts of isosorbide dinitrate to the pressure-sensitive adhesive solution per 80 parts of the solid component of the solution, without adding either of isopropyl myristate and a crosslinking agent.

COMPARATIVE EXAMPLE 17

A medical preparation for percutaneous absorption was obtained in the same manner as in Example 10 except that a coating composition for forming a pressure-sensitive adhesive layer was prepared by adding, to the pressure-sensitive adhesive solution prepared in Comparative Example 15, 20 parts of isosorbide dinitrate and 8 parts of isopropyl myristate per 72 parts of the solid component of the solution.

COMPARATIVE EXAMPLE 18

A medical preparation for percutaneous absorption in which the pressure-sensitive adhesive layer did not contain dissolution-controlling agent and had not been crosslinked was obtained in the same manner as in Example 11 except that a coating composition for forming a pressure-sensitive adhesive layer was prepared by dissolving 7 parts of ketoprofen in the pressure-sensitive adhesive solution prepared in Comparative Example 15 per 93 parts of the solid component of the solution.

Each of the medical preparations for percutaneous absorption, obtained in Examples 10 and 11 and Comparative Examples 16 to 18, was cut into 7.1 cm x 7.1 cm, subsequently allowed to stand at room temperature for 24 hours, and then applied to a rabbit on its back part which had been depilated. After the application, a blood sample was taken from the rabbit at intervals of a predetermined period of time. The concentrations of isosorbide dinitrate or ketoprofen in the blood samples were determined by gas chromatography. The results obtained are shown in Table 3.

Further, the medical preparations were also evaluated for skin-stimulating properties, and the order of the degree of stimulation to the skin was as follows.

Comparative Example 18 ≥ Comparative Example 16 ≥ Comparative Example 17 > Example 10 ≥ Example 11

TABLE 3

|  | Maximum drug concentration in blood (ng/ml) | Time required to reach the maximum drug concentration (hr) | Area of the region beneath the drug-in-blood concentration curve (ng • hr/ml) |
|---|---|---|---|
| Example 10 | 260 | 2.5 | 1480 |
| Example 11 | 2040 | 2.0 | 9880 |
| Comparative Example 16 | 108 | 2.5 | 620 |
| Comparative Example 17 | 121 | 2.5 | 710 |
| Comparative Example 18 | 830 | 2.0 | 3250 |

EXAMPLE 12

60 Parts of an acrylic ester-based polymer obtained by copolymerizing 97 parts of 2-ethylhexyl acrylate and 3 parts of acrylic acid in ethyl acetate in an inert gas atmosphere was mixed with 40 parts of octyl palmitate. Thereto was then added 0.25 part of a trifunctional isocyanate per 99.75 parts of the acrylic ester-based polymer. Thus, a pressure-sensitive adhesive solution was prepared.

The pressure-sensitive adhesive solution obtained was coated on a polyester separator at a dry thickness of 60 $\mu$m, and the coating was dried to form a pressure-sensitive adhesive layer.

The thus-obtained pressure-sensitive adhesive layer was transferred to a polyester film (9 $\mu$m thick) as substrate on one side, thereby obtaining a gel material according to the present invention.

EXAMPLE 13

The same procedures as in Example 12 were conducted except that a pressure-sensitive adhesive solution was prepared by mixing 45 parts of the acrylic ester-based polymer prepared in Example 14, 35 parts of octyl palmitate, and 20 parts of isosorbide dinitrate. Thus, a gel-based medical preparation according to the present invention was obtained.

EXAMPLE 14

A gel-based medical preparation according to the present invention was obtained in the same manner as in Example 13 except that 1,3-butanediol was used in place of octyl palmitate.

COMPARATIVE EXAMPLE 19

A gel material was obtained in the same manner as in Example 12 except that the acrylic ester-based polymer prepared in Example 12 was not crosslinked (i.e., the trifunctional isocyanate was not added).

This gel material showed considerable cohesive failure, so that it was unable to be evaluated in any of the tests which will be described after.

COMPARATIVE EXAMPLE 20

A gel material was obtained in the same manner as in Example 12 except that octyl palmitate was not added.

COMPARATIVE EXAMPLE 21

The same procedures as in Example 12 were conducted except that a pressure-sensitive adhesive solution was prepared by adding 20 parts of isosorbide dinitrate as a drug to the acrylic ester-based polymer, without adding either of octyl palmitate and a tri-functional isocyanate. Thus, a gel-based medical preparation was obtained.

EXAMPLE 15

50 Parts of an acrylic ester-based polymer obtained by copolymerizing 70 parts of isononyl acrylate, 25 parts of N-vinyl-2-pyrrolidone, and 5 parts of acrylic acid in ethyl acetate in an inert gas atmosphere was mixed with 50 parts of isotridecyl myristate. Thereto was then added 0.3 part of (ethyl acetoacetate)titanium diisopropylate per 99.7 parts of the acrylic ester-based polymer. Thus, a pressure-sensitive adhesive solution was prepared.

The pressure-sensitive adhesive solution obtained was coated on a separator at a dry thickness of 60 $\mu$m, and the coating was dried to form a pressure-sensitive adhesive layer. This pressure-sensitive adhesive layer was transferred to a substrate film which was a laminate of a polyester nonwoven fabric (basis weight 8 g/m$^2$) and a polyester film (3 $\mu$m thick), on the nonwoven fabric side. Thus, a gel material according to the present invention was obtained.

EXAMPLE 16

The same procedures as in Example 15 were conducted except that a pressure-sensitive adhesive solution was prepared by mixing 47 parts of the acrylic ester-based polymer prepared in Example 15, 50 parts of isotridecyl myristate, and 3 parts of estradiol. Thus, a gel-based medical preparation according to the present invention was obtained.

EXAMPLE 17

A gel-based medical preparation according to the present invention was obtained in the same manner as in Example 16 except that ethyl oleate was used in place of isotridecyl myristate.

COMPARATIVE EXAMPLE 22

A gel material was obtained in the same manner as in Example 15 except that the acrylic ester-based polymer prepared in Example 15 was not crosslinked (i.e., (ethyl acetoacetate)titanium diisopropylate was not added).

This gel material showed considerable cohesive failure, so that it was unable to be evaluated in any of the tests which will be described after.

COMPARATIVE EXAMPLE 23

A gel material was obtained in the same manner as in Example 15 except that isotridecyl myristate was not added.

COMPARATIVE EXAMPLE 24

The same procedures as in Example 15 were conducted except that neither isotridecyl myristate or (ethyl acetoacetate)titanium diisopropylate was added to the pressure-sensitive adhesive solution and that 3 parts of estradiol was added to the pressure-sensitive adhesive solution. Thus, a gel-based medical preparation was obtained.

Each of the gel materials and gel preparations obtained in Examples 12 to 17 and Comparative Examples 20, 21, 23, and 24 was subjected to the following tests. The results obtained are shown in Table 4.

[Adhesion Strength to Bakelite]

Each sample which had been cut into a 12 mm-wide strip was placed on a Bakelite plate and bonded thereto by moving a 850 g roller forward and backward once on the strip. The strip was then peeled from the plate in the 180° direction at a rate of 300 mm/min, and the peel force required for the peeling was measured.

[Degree of Pain]

Each sample which had been cut into a size of 5 cm$^2$ was applied to 5 volunteers at their upper arm inner parts. One hour later, the sample was stripped and the degree of a pain caused by the stripping was evaluated based on the following criteria, in terms of average value.

1: No pain, 2: Very slight pain, 3: Slight pain, 4: Pain, 5: Severe pain.

[Amount of Peeled Horny Layer Tissue]

Each sample which had been cut into a strip 12 mm wide and 50 mm long was applied to 5 volunteers at their lower arm inner parts and kept being applied thereto for 6 hours. Thereafter, the test sample was stripped, subsequently immersed for 24 hours in a dyeing solution having the composition shown below, and then washed with distilled water, thereby dyeing horny layer cells that had peeled from the skin.

| Composition of Dyeing Solution: | |
|---|---|
| Gentian violet | 1.0% |
| Brilliant green | 0.5% |
| Distilled water | 98.5% |

Each of the thus-dyed test samples was cut into 12 mm x 5 mm and then immersed in 5 ml of a 1% aqueous solution of sodium dodecyl sulfate over a whole day and night, thereby extracting the dyes from the horny layer cells adhered to the sample. The absorbance of this extract (595 nm) was measured with a spectrophotometer. As a control, each of samples which had not been applied to the skin was subjected to the same extraction treatment. The absorbance was determined from a difference spectrum with the above test sample. Using the absorbance values obtained, the samples were compared in the number of horny layer cells, based on the assumption that the higher the absorbance measured, the larger the amount of peeled horny layer cells.

It is noted that there was a good correlation between the number of peeled horny layer cells counted with a stereomicroscope and the absorbance measured as described above.

[Adhesion Strength to Skin (Interfacial Adhesion Strength)]

Each sample which had been cut into a strip 12 mm wide and 50 mm long was applied to 5 volunteers at their lower arm inner parts and kept being applied thereto for six hours. Thereafter, the sample was stripped from the skin in the 180° direction at a rate of 100 mm/min and the stripping force required for the stripping was measured.

In this test, stripping of the samples of the Examples caused substantially no peeling of horny layer cells and, hence, those adhesion strength values for the Examples which are given in Table 4 mean values of the adhesion strength referred to as interfacial adhesion strength in this invention. On the other hand, stripping of the samples of the Comparative Examples resulted in peeling of horny layer cells, and it is apparent that the bonding strength among horny layer cells was lower than the interfacial adhesion strengths of the comparative samples. Thus, the adhesion strength values for the Comparative Examples mean values of the bonding strength among horny layer cells.

TABLE 4

| | Adhesion to Bakelite (g) | Degree of pain | Amount of peeled horny layer tissue (abs.) | Adhesion to skin (g) |
|---|---|---|---|---|
| Example 12 | 105 | 1.0 | 0.4566 | 20 |
| Example 13 | 131 | 1.0 | 0.5412 | 22 |
| Example 14 | 128 | 1.0 | 0.2118 | 23 |
| Example 15 | 163 | 1.0 | 0.4041 | 27 |
| Example 16 | 157 | 1.0 | 0.3240 | 19 |
| Example 17 | 176 | 1.0 | 0.4064 | 21 |
| Comparative Example 20 | 578 | 4.0 | 1.4329 | 35 |
| Comparative Example 21 | 496 | 3.0 | 1.3044 | 48 |
| Comparative Example 23 | 760 | 4.0 | 1.4731 | 39 |
| Comparative Example 24 | 699 | 3.0 | 1.1014 | 37 |

EXAMPLE 18

50 Parts of an acrylic ester-based polymer obtained by copolymerizing 95 parts of 2-ethylhexyl acrylate and 5 parts of acrylic acid in ethyl acetate was mixed with 50 parts of isopropyl myristate. To this base solution was added, in ethyl acetate, 0.15 part of a trifunctional isocyanate (Coronate HL, manufactured by Nippon Polyurethane Industry Co., Ltd.) per 99.85 parts of the solid component of the solution. Thus, a pressure-sensitive adhesive solution was prepared.

The solution obtained was coated on a polyester separator at a dry thickness of 60 $\mu$m, and the coating was dried to form a crosslinked pressure-sensitive adhesive layer. This pressure-sensitive adhesive layer was transferred to a substrate film which was a laminate of a polyester nonwoven fabric (12 g/m$^2$) and a polyester film (2 $\mu$m thick), on the nonwoven fabric side. The resulting structure was aged at 70°C for 48 hours to obtain an acrylic pressure-sensitive sheet according to the present invention.

EXAMPLE 19

An acrylic pressure-sensitive sheet according to the present invention was obtained in the same manner as in Example 18 except that the thickness of the pressure-sensitive adhesive layer was changed to 100 $\mu$m.

EXAMPLE 20

An acrylic pressure-sensitive sheet according to the present invention was obtained in the same manner as in Example 18 except that isotridecyl myristate was used in place of isopropyl myristate.

EXAMPLE 21

An acrylic pressure-sensitive sheet according to the present invention was obtained in the same manner as in Example 18 except that a base solution to which the crosslinking agent was added was prepared by mixing 70 parts of the acrylic ester-based polymer and 30 parts of isopropyl myristate.

EXAMPLE 22

An acrylic pressure-sensitive sheet according to the present invention was obtained in the same manner as in Example 18 except that a base solution to which the crosslinking agent was added was prepared by mixing 40 parts of the acrylic ester-based polymer and 60 parts of isopropyl myristate.

EXAMPLE 23

The same procedures as in Example 18 were conducted except that a base solution to which the crosslinking agent was added was prepared by mixing 40 parts of the acrylic ester-based polymer, 40 parts of isopropyl myristate, and 20 parts of isosorbide dinitrate. Thus, a pressure-sensitive medical preparation according to the present invention was obtained.

EXAMPLE 24

The same procedures as in Example 20 were conducted except that a base solution to which the crosslinking agent was added was prepared by mixing 30 parts of the acrylic ester-based polymer, 50 parts of isotridecyl myristate, and 20 parts of isosorbide dinitrate. Thus, a pressure-sensitive medical preparation according to the present invention was obtained.

EXAMPLE 25

50 Parts of an acrylic ester-based polymer obtained by copolymerizing 72 parts of 2-ethylhexyl acrylate, 3 parts of acrylic acid, and 25 parts of N-vinyl-2-pyrrolidone in ethyl acetate was mixed with 50 parts of isopropyl myristate. To this base solution was added, in ethyl acetate, 0.3 part of (ethyl acetoacetate)aluminum diisopropylate per 99.7 parts of the solid component of the solution. Thus, a pressure-sensitive adhesive solution was prepared. Using this solution, an acrylic pressure-sensitive sheet according to the present invention was obtained in the same manner as in Example 20.

EXAMPLE 26

The same procedures as in Example 25 were conducted except that a base solution to which the cross-linking agent was added was prepared by mixing 45 parts of the acrylic ester-based polymer prepared in Example 25, 40 parts of isopropyl myristate, and 15 parts of nifedipine. Thus, a pressure-sensitive medical preparation according to the present invention was obtained.

EXAMPLE 27

A pressure-sensitive medical preparation according to the present invention was obtained in the same manner as in Example 26 except that a base solution to which the crosslinking agent was added was prepared by mixing 35 parts of the acrylic ester-based polymer, 50 parts of isopropyl myristate, and 15 parts of nifedipine.

COMPARATIVE EXAMPLE 25

An acrylic pressure-sensitive sheet was obtained in the same manner as in Example 18 except that only the acrylic ester-based polymer prepared in Example 18 was used to form a pressure-sensitive adhesive layer.

COMPARATIVE EXAMPLE 26

An acrylic pressure-sensitive sheet was obtained in the same manner as in Example 18 except that isopropyl myristate was not added.

COMPARATIVE EXAMPLE 27

An acrylic pressure-sensitive sheet was obtained in the same manner as in Example 18 except that a base solution to which the crosslinking agent was added was prepared by mixing 85 parts of the acrylic ester-based polymer and 15 parts of isopropyl myristate.

COMPARATIVE EXAMPLE 28

An acrylic pressure-sensitive sheet was obtained in the same manner as in Example 18 except that a base solution to which the crosslinking agent was added was prepared by mixing 30 parts of the acrylic ester-based polymer and 70 parts of isopropyl myristate. The pressure-sensitive adhesive layer in the thus-obtained pressure-sensitive sheet had poor cohesive force, so that after stripping of the sheet from the skin, part of the pressure-sensitive adhesive remained on the skin. Further, the sheet also had very poor application feeling.

COMPARATIVE EXAMPLE 29

An acrylic pressure-sensitive sheet was obtained in the same manner as in Example 18 except that the trifunctional isocyanate as a crosslinking agent was not added. This pressure-sensitive sheet showed considerable cohesive failure, so that it was unable to be evaluated in the tests described after.

COMPARATIVE EXAMPLE 30

The same procedures as in Example 18 were conducted except that a pressure-sensitive adhesive solution was prepared by mixing 80 parts of the acrylic ester-based polymer and 20 parts of isosorbide dinitrate, without adding either of isopropyl myristate and a trifunctional isocyanate. Thus, a pressure-sensitive medical preparation was obtained.

COMPARATIVE EXAMPLE 31

A pressure-sensitive medical preparation was obtained in the same manner as in Example 23 except that a base solution to which the crosslinking agent was added was prepared by mixing 65 parts of the acrylic ester-based polymer, 15 parts of isopropyl myristate, and 20 parts of isosorbide dinitrate.

COMPARATIVE EXAMPLE 32

An acrylic pressure-sensitive sheet was obtained in the same manner as in Example 25 except that only the acrylic ester-based polymer prepared in Example 25 was used to form a pressure-sensitive adhesive layer.

COMPARATIVE EXAMPLE 33

The same procedures as in Example 25 were conducted except that a base solution to which the crosslinking agent was added was prepared by mixing 85 parts of the acrylic ester-based polymer and 15 parts of nifedipine. Thus, a pressure-sensitive medical preparation was obtained.

COMPARATIVE EXAMPLE 34

The same procedures as in Example 25 were conducted except that a base solution to which the cross-linking agent was added was prepared by mixing 70 parts of the acrylic ester-based polymer, 15 parts of isopropyl myristate, and 15 parts of nifedipine. Thus, a pressure-sensitive medical preparation was obtained.

Each of the pressure-sensitive sheet and pressure-sensitive medical preparation samples obtained in Examples 18 to 27 and Comparative Examples 25 to 34 given above was stored at 40°C under high-humidity conditions of 75% for 2 weeks and then subjected to the following tests. In the samples which were to be subjected to the test for measuring the amount of peeled horny layer tissues, a single-layer film (9 $\mu$m thick) not laminated with a nonwoven fabric was employed as the substrate, because use of a nonwoven fabric makes accurate measurement difficult due to absorption of the dyeing solution by the nonwoven fabric. The results obtained are shown in Table 5.

[Application to Rabbit]

Each sample was cut into a size of 50 cm$^2$ and then applied to a rabbit on its back part which had been depilated beforehand. At each of 1, 2, 4, 6, and 8 hours after the application, 2 ml of a blood sample was taken from the rabbit. The drug concentrations in these blood samples were determined by gas chromatography.

[Adhesion Strength Test]

Each sample which had been cut into a 12 mm-wide strip was placed on a Bakelite plate and bonded thereto by moving a 300 g roller forward and backward once on the strip. The strip was then peeled from the plate in the 180° direction at a rate of 300 mm/min, and the peel force required for the peeling was measured.

[Shear Deformation Test]

Measurement was made using a Yamamoto-type cohesive force-measuring device (refer to Seimitsu Kohgaku Kaishi, 52, (1), 894 (1986) and The 23rd Setchaku Kenkyu Happyo-kai Yohshi-shu, 44 (1985)).

Each sample was applied to a stainless-steel adherend (5 mm wide) in an adhesion area of 5 mm x 25 mm. This sample was allowed to stand for 30 minutes, while the same load was kept being laid upon both ends of the sample. Thereafter, a shearing stress was imposed on the sample by applying a 40 g/cm$^2$ load to one end of the sample, and the amount of a shift (shift completion distance) caused by the shearing stress was measured.

[Pain by Stripping]

Each sample was applied to 5 volunteers at their upper arm inner parts. Two hours later, the sample was stripped and the degree of a pain caused by the stripping was evaluated. The evaluation was made in 5 grades with samples that caused the slightest pain being rated as 1, and the rating values for each example were averaged. As a control, the samples of Comparative Example 25 were rated as 5.

[Amount of Peeled Horny Layer Tissue]

Each sample which had been cut into a disk shape having a diameter of 16 mm was applied to 3 volunteers at their upper arm inner parts and kept being bonded thereto for 2 hours. Thereafter, the sample was stripped, subsequently immersed for 3 minutes in a dyeing solution (Gentian violet 1%, Brilliant green 0.5%, distilled water 98.5%), and then washed with water, thereby dyeing horny layer cells adhered to the sample surface.

The resulting samples were immersed in a 5% aqueous solution of sodium dodecyl sulfate over a whole day and night, thereby extracting the dyes. By measuring the absorbances of these extracts (595 nm), the samples were compared in the number of horny layer cells peeled from the skin, based on the assumption that the higher the absorbance, the larger the amount of peeled horny layer cells.

It is noted that there was a good correlation between the number of peeled horny layer cells counted with a stereomicroscope and the absorbance measured as described above.

TABLE 5

| | Adhesion strength (g) | Pain | Amount of peeled horny layer cells (abs.) | Maximum drug concentration in blood (ng/ml) | Time required to reach the maximum drug concentration (hr) | Shear-shift completion distance ($\mu$m) |
|---|---|---|---|---|---|---|
| Example 18 | 65 | 1.3 | 0.330 | - | - | 150 |
| Example 19 | 98 | 1.4 | 0.375 | - | - | 300 |
| Example 20 | 73 | 1.3 | 0.374 | - | - | 130 |
| Example 21 | 102 | 1.6 | 0.376 | - | - | 80 |
| Example 22 | 52 | 1.2 | 0.412 | - | - | 210 |
| Example 23 | 68 | 1.2 | 0.382 | 263 | 2.5 | 100 |
| Example 24 | 60 | 1.2 | 0.339 | 258 | 2.5 | 170 |
| Example 25 | 83 | 1.4 | 0.355 | - | - | 120 |
| Example 26 | 99 | 1.4 | 0.397 | 148 | 4.0 | 160 |
| Example 27 | 118 | 1.6 | 0.402 | 162 | 4.0 | 220 |
| Comparative Example 25 | 420 | 5.0 | 1.400 | - | - | Measurement impossible |
| Comparative Example 26 | 395 | 5.0 | 1.550 | - | - | 10 |
| Comparative Example 27 | 594 | 5.0 | 1.150 | - | - | 30 |
| Comparative Example 28 | 40 | 1.2 | 0.342 | - | - | 360 |
| Comparative Example 29 | - | - | - | - | - | - |
| Comparative Example 30 | 311 | 4.0 | 1.413 | 106 | 2.5 | Measurement impossible |
| Comparative Example 31 | 521 | 5.0 | 1.207 | 129 | 2.5 | 20 |
| Comparative Example 32 | 418 | 5.0 | 1.523 | - | - | Measurement impossible |
| Comparative Example 33 | 396 | 5.0 | 1.603 | 25 | 4.0 | 8 |
| Comparative Example 34 | 613 | 5.0 | 1.037 | 69 | 4.0 | Measurement impossible |

EXAMPLE 28

In an inert gas atmosphere, 95 parts of 2-ethylhexyl acrylate and 5 parts of acrylic acid were copolymerized in 50 parts of ethyl acetate, thereby preparing a solution of an acrylic ester-based polymer.

To this solution was added 48.2 parts of isopropyl myristate and 0.07 part of a trifunctional isocyanate (trade name, Coronate HL; manufactured by Nippon Polyurethane Industry Co., Ltd.) per 51.8 parts of the polymer in the solution.

This solution was coated on a 75 $\mu$m-thick polyester separator on the liner side using a doctor blade at a dry thickness of 60 $\mu$m, and the coating was dried at 100°C for 5 minutes. The resulting pressure-sensitive adhesive layer was bonded with a substrate which was a laminate of a polyester nonwoven fabric (12 g/m$^2$) and a 2 $\mu$m-thick polyester film,

thereby obtaining a pressure-sensitive sheet.

COMPARATIVE EXAMPLE 35

A pressure-sensitive sheet was obtained in the same manner as in Example 28 except that the isopropyl myristate and trifunctional isocyanate were not added and the acrylic ester-based polymer solution only was used to form a pressure-sensitive adhesive layer.

COMPARATIVE EXAMPLE 36

A pressure-sensitive sheet was obtained in the same manner as in Example 28 except that a pressure-sensitive adhesive layer was formed without adding a trifunctional isocyanate. This pressure-sensitive sheet showed considerable cohesive failure, so that it was unable to be evaluated in any of the tests described after.

EXAMPLE 29

The same procedures as in Example 28 were conducted except that a coating solution for forming a pressure-sensitive adhesive layer was prepared by adding, to the acrylic ester-based polymer solution, 40 parts of isopropyl myristate, 17 parts of isosorbide dinitrate, and 0.06 part of the trifunctional isocyanate per 43 parts of the polymer in the solution. Thus, a pressure-sensitive medical preparation was obtained.

EXAMPLE 30

A pressure-sensitive medical preparation was obtained in the same manner as in Example 29 except that 0.09 part of (ethyl acetoacetate)aluminum diisopropylate was used in place of 0.06 part of the trifunctional isocyanate.

EXAMPLE 31

In an inert gas atmosphere, 75 parts of 2-ethylhexyl acrylate, 22 parts of N-vinyl-2-pyrrolidone, and 3 parts of acrylic acid were copolymerized in 150 parts of ethyl acetate, thereby preparing a solution of an acrylic ester-based polymer. To this solution was added 40 parts of octyl palmitate and 0.12 part of a trifunctional isocyanate per 100 parts of the polymer in the solution. Thus, a viscous solution was obtained. Using this viscous solution as a coating solution, a pressure-sensitive sheet was obtained in the same manner as in Example 28.

COMPARATIVE EXAMPLE 37

A pressure-sensitive sheet was obtained in the same manner as in Example 31 except that the amount of the trifunctional isocyanate was changed to 0.6 part.

COMPARATIVE EXAMPLE 38

A pressure-sensitive sheet was obtained in the same manner as in Example 31 except that the amount of the trifunctional isocyanate was changed to 0.03 part.

EXAMPLE 32

The same procedures as in Example 31 were conducted except that a coating solution for forming a pressure-sensitive adhesive layer was prepared by adding, to the acrylic ester-based polymer solution, 40 parts of octyl palmitate, 2.5 parts of estradiol, and 0.115 part of the trifunctional isocyanate per 57.5 parts of the polymer in the solution. Thus, a pressure-sensitive medical preparation was obtained.

Evaluation Tests:

Each of the samples obtained in Examples 28 to 32 and Comparative Examples 35 to 38 given above was stored at 70°C for 2 days and then subjected to the following tests.

[Adhesion Strength to the Same Pressure-Sensitive Layer]

Two test pieces of the same sample, which had been cut into 12 mm-wide strips and from which the separator had been removed, were superposed on each other with the pressure-sensitive adhesive layers facing each other. The superposed test pieces were bonded to each other by moving a 850 g roller forward and backward once on the test pieces. The thus-bonded test pieces were then peeled in the 180° direction at a rate of 300 mm/min to measure the adhesion strength.

The above adhesion strength measurement was made using a Schopper tensile tester (manufactured by Ueshima Seisakusho, Japan).

[Adhesion to Skin]

Each sample was applied to 6 volunteers at their upper arm inner parts, and 24 hours later, the state of each applied sample (adhesion to the skin) was visually evaluated. The evaluation was made in 5 grades, with samples best adhered to the skin being rated as 5 and samples in which considerable part thereof had peeled off the skin being rated as 1.

[Skin Stimulation (Pain) during Stripping]

After the above test for examining adhesion to the skin, each sample was stripped from the skin and the stimulation (pain) caused to the skin by the stripping was evaluated. The evaluation was made in 5 grades with samples that gave no stimulation to the skin being rated as 1, and the rating values for each example were averaged. As a control, the samples of Comparative Example 35 were rated as 5.

TABLE 6

| | Adhesion strength (g/12 mm-width) | Adhesion to skin (n=6) | Skin stimulation (Pain) (n=t) |
|---|---|---|---|
| Example 28 | 38 | 4.8 | 1.2 |
| Example 29 | 54 | 5.0 | 1.0 |
| Example 30 | 55 | 4.8 | 1.3 |
| Example 31 | 133 | 4.7 | 1.5 |
| Example 32 | 121 | 5.0 | 1.2 |
| Comparative Example 35 | 435 | 4.0 | 5.0 |
| Comparative Example 36 | - | - | - |
| Comparative Example 37 | 12 | 1.3 | 1.2 |
| Comparative Example 38 | 338 | 3.8 | 4.3 |

As is apparent from Table 6, the pressure-sensitive sheets and pressure-sensitive medical preparations according to the present invention showed good adhesion to the skin and, when stripped from the skin, caused almost no physical stimulation (pain) to the skin.

**Claims**

1. An acrylic ester gel material for application to the skin, comprising a substrate, and a chemically crosslinked pressure-sensitive adhesive layer formed on at least one side of said substrate, said crosslinked pressure-sensitive adhesive layer comprising: an acrylic ester-based polymer comprising a (meth)acrylic acid alkyl ester; and an organic liquid component for plasticizing said polymer to impart softness to said pressure-sensitive adhesive layer, said organic liquid component being compatible with said polymer and being at least one organic liquid selected from the group consisting of an organic solvent, a surfactant, a plasticizer, a hydrocarbon, and a fatty acid ester, said pressure-sensitive adhesive layer being chemically crosslinked so as to provide a gel content in said chemically crosslinked pressure-sensitive adhesive layer of from 50 to 80% by weight of said polymer.

2. An acrylic ester gel material for application to the skin as claimed in claim 1, wherein the chemical crosslinking is conducted using a crosslinking agent selected from the group consisting of a titanium-containing alcoholate, an aluminum-containing alcoholate, a titanium-containing chelate, an aluminum-containing chelate and a trifunctional isocyanate.

3. An acrylic ester gel material for application to the skin as claimed in claim 2, wherein said chemical crosslinking agent is used in an amount of from 0.05 to 3.0 parts by weight per 100 parts by weight of the acrylic ester-based polymer.

4. An acrylic ester gel material for application to the skin as claimed in claim 1, wherein said acrylic ester-based polymer is a copolymer obtained by copolymerizing from 60 to 98% by weight of said (meth)acrylic acid alkyl ester having from 4 to 15 carbon atoms in the alkyl moiety and from 2 to 40% by weight of a monomer copolymerizable with said (meth)acrylic acid alkyl ester.

5. An acrylic ester gel material for application to the skin as claimed in claim 4, wherein said copolymerizable monomer is at least one monomer selected from the group consisting of a carboxyl group-containing monomer, a hydroxyl group-containing acrylate, an amide group-containing acrylate, an aminoalkyl group-containing acrylate, an alkoxyl group-containing acrylate and a vinyl monomer.

6. An acrylic ester gel material for application to the skin as claimed in claim 1, wherein the amount of said organic liquid component contained in the pressure-sensitive adhesive layer is from 25 to 200 parts by weight per 100 parts by weight of the acrylic ester-based polymer.

7. An acrylic ester gel material for application to the skin as claimed in claim 6, wherein the amount of said organic liquid component contained in the pressure-sensitive adhesive layer is form 40 to 160 parts by weight per 100 parts by weight of the acrylic ester-based polymer.

8. An acrylic ester gel material for application to the skin as claimed in claim 1, wherein said pressure-sensitive adhesive layer has an adhesive strength when bonded to a second pressure-sensitive adhesive layer of identical composition of from 20 to 180 g/12mm-width, as measured by a 180° constant rate peel strength test.

9. An acrylic ester gel material as claimed in claim 1, wherein said pressure-sensitive adhesive layer has a shear-deformation-shift completion distance when a shearing stress is imposed on said pressure-sensitive adhesive layer of from 0.5 to 6 times the thickness of said pressure-sensitive adhesive layer.

10. An organic ester gel material for application to the skin as claimed in claim 1, wherein said pressure-sensitive adhesive layer has an interfacial adhesion strength between said pressure-sensitive adhesive layer and horney layer cells substantially lower than the bonding strength among said horny layer cells.

11. An acrylic ester gel-based medical preparation for percutaneous absorption which comprises:

a substrate, and a chemically crosslinked pressure-sensitive adhesive layer formed on at least one side of said substrate, said crosslinked pressure-sensitive adhesive layer comprising: an acrylic ester-based polymer comprising a (meth)acrylic acid alkyl ester; and an organic liquid component for plasticizing said polymer to impart softness to said pressure-sensitive adhesive layer, said organic liquid component being compatible with said polymer and being at least one organic liquid selected from the group consisting of an organic solvent, a surfactant, a plasticizer, a hydrocarbon, and a fatty acid ester, said pressure-sensitive adhesive layer being chemically crosslinked so as to provide a gel content in said chemically crosslinked pressure-sensitive adhesive layer of form 50 to 80% by weight of said polymer; and
a drug contained in the pressure-sensitive adhesive layer.

12. An acrylic ester gel-based medical preparation as claimed in claim 11, wherein said drug is present in an amount of from 1 to 40% by weight based on the weight of the pressure-sensitive adhesive layer.

13. An acrylic ester gel-based medical preparation as claimed in claim 11, wherein said drug has a solubility in said pressure-sensitive adhesive layer of at least 4% by weight.

14. An acrylic ester gel-based medical preparation as claimed in claim 11, wherein said drug has a solubility in said organic liquid component of up to 3% by weight.

15. An acrylic ester gel-based medical preparation as claimed in claim 11, wherein said pressure-sensitive adhesive layer has an adhesive strength when bonded to a second pressure-sensitive adhesive layer of from 20 to 180 g/12mm-width, as measured by a 180° constant rate peel strength test.

16. An acrylic ester gel-based medical preparation as claimed in claim 11, wherein said pressure-sensitive adhesive layer has a shear-deformation-shift completion distance when a shearing stress is imposed on said pressure-sensitive adhesive layer of from 0.5 to 6 times the thickness of said pressure-sensitive adhesive layer.

17. An acrylic ester gel-based medical preparation as claimed in claim 11, wherein said pressure-sensitive adhesive layer has an interfacial adhesion strength between said pressure-sensitive adhesive layer and horney layer cells substantially lower than the bonding strength among said horny layer cells.

**Patentansprüche**

1. Acrylsäureester-Gelmaterial zum Aufbringen auf die Haut, welches umfaßt: ein Substrat und eine chemisch vernetzte druckempfindliche Haftschicht, die auf wenigstens einer Seite des Substrats gebildet ist, wobei die vernetzte druckempfindliche Haftschicht ein Polymer auf Acrylsäureester-Basis, welches einen (Meth)acrylsäurealkylester umfaßt, und einen organischen flüssigen Bestandteil zum Weichmachen des Polymers umfaßt, um der druckempfindlichen Haftschicht Weichheit zu verleihen, wobei der organische flüssige Bestandteil mit dem Polymer verträglich ist und wenigstens eine organische Flüssigkeit ist, die gewählt ist aus der aus organischem Lösungsmittel, oberflächenaktivem Mittel, Weichmacher, Kohlenwasserstoff und Fettsäureester bestehenden Gruppe, wobei die druckempfindliche Haftschicht derart chemisch vernetzt ist, daß ein Gelgehalt von 50 bis 80 Gew.-% des Polymers in der chemisch vernetzten druckempfindlichen Haftschicht geschaffen wird.

2. Acrylsäureester-Gelmaterial zum Aufbringen auf die Haut nach Anspruch 1, worin die chemische Vernetzung unter Verwendung eines Vernetzungsmittels durchgeführt wird, das gewählt ist aus der Gruppe, die besteht aus einem Titan enthaltenden Alkoholat, einem Aluminium enthaltenden Alkoholat, einem Titan enthaltenden Chelat, einem Aluminium enthaltenden Chelat und einem trifunktionellen Isocyanat.

3. Acrylsäureester-Gelmaterial zum Aufbringen auf die Haut nach Anspruch 2, worin das chemische Vernetzungsmittel in einer Menge von 0,05 bis 3,0 Gew.-Teilen pro 100 Gew.-Teile des Polymers auf Acrylsäureester-Basis verwendet wird.

4. Acrylsäureester-Gelmaterial zum Aufbringen auf die Haut nach Anspruch 1, worin das Polymer auf Acrylsäureester-Basis ein Copolmyer ist, das durch Copolmyerisation von 60 bis 98 Gew.-% (Meth)acrylsäurealkylester mit 4 bis 15 Kohlenstoffatomen im Alkylteil und 2 bis 40 Gew.-% eines Monomers erhalten wird, das mit dem (Meth)acrylsäurealkylester copolymerisierbar ist.

5. Acrylsäureester-Gelmaterial zum Aufbringen auf die Haut nach Anspruch 4, worin das copolymerisierbare Monomer wenigstens ein Monomer ist, das gewählt ist aus der Gruppe, die besteht aus einem eine Carboxy-Gruppe enthaltenden Monomer, einem eine Hydroxy-Gruppe enthaltenden Acrylat, einem eine Amid-Gruppe enthaltenden Acrylat, einem eine Aminoalkyl-Gruppe enthaltenden Acrylat, einem eine Alkoxy-Gruppe enthaltenden Acrylat und einem Vinylmonomer.

6. Acrylsaureester-Gelmaterial zum Aufbringen auf die Haut nach Anspruch 1, worin die Menge des organischen flüssigen Bestandteils, der in der druckempfindlichen Haftschicht enthalten ist, im Bereich von 25 bis 200 Gew.-Teilen pro 100 Gew.-Teile des Polymers auf Acrylsäureester-Basis liegt.

7. Acrylsäureester-Gelmaterial zum Aufbringen auf die Haut nach Anspruch 6, worin die Menge des organischen flüssigen Bestandteils, der in der druckempfindlichen Haftschicht enthalten ist, im Bereich von 40 bis 160 Gew.-Teilen pro 100 Gew.-Teile des Polymers auf Acrylsäureester-Basis liegt.

8. Acrylsäureester-Gelmaterial zum Aufbringen auf die Haut nach Anspruch 1, worin die druckempfindliche Haftschicht eine Haftfestigkeit von 20 bis 180 g/12 mm Breite aufweist, gemessen mit einem Test zur Bestimmung der Abschalfestigkeit unter einem Winkel von 180° bei konstanter Geschwindigkeit, nachdem diese auf eine zweite druckempfindliche Haftschicht identischer Zusammensetzung aufgebracht wurde.

9. Acrylsäureester-Gelmaterial nach Anspruch 1, worin die druckempfindliche Haftschicht einen bei Scherungsbeanspruchung auftretenden Distanzwert der vollständigen Verschiebung des 0,5- bis 6-fachen der Dicke der druckempfindlichen Haftschicht aufweist, wenn eine Scherungsbeanspruchung auf die druckempfindliche Haftschicht aufgebracht wird.

**10.** Organisches Ester-Gelmaterial zum Aufbringen auf die Haut nach Anspruch 1, worin die druckempfindliche Haftschicht eine Grenzflächen-Haftfestigkeit zwischen der druckempfindlichen Haftschicht und den Hornschicht-Zellen aufweist, die wesentlich niedriger ist als die Bindungsfestigkeit zwischen den Homschicht-Zellen.

**11.** Medizinische Zubereitung auf Acrylsäureestergel-Basis zur perkutanen Absorption, welche umfaßt:

- ein Substrat und eine chemisch vernetzte druckempfindliche Haftschicht, die auf wenigstens einer Seite des Substrats gebildet ist, wobei die vernetzte druckempfindliche Haftschicht ein Polymer auf Acrylsäureester-Basis, welches einen (Meth)acrylsäurealkylester umfaßt, und einen organischen flüssigen Bestandteil zum Weichmachen des Polymers umfaßt, um der druckempfindlichen Haftschicht Weichheit zu verleihen, wobei der organische flüssige Bestandteil mit dem Polymer verträglich ist und wenigstens eine organische Flüssigkeit ist, die gewählt ist aus der aus organischem Lösungsmittel, oberflächenaktivem Mittel, Weichmacher, Kohlenwasserstoff und Fettsäureester bestehenden Gruppe, wobei die druckempfindliche Haftschicht derart chemisch vernetzt ist, daß ein Gelgehalt von 50 bis 80 Gew.-% des Polymers in der chemisch vernetzten druckempfindlichen Haftschicht geschaffen wird; und
- ein in der druckempfindlichen Haftschicht enthaltenes Medikament.

**12.** Medizinische Zubereitung auf Acrylsäureestergel-Basis nach Anspruch 11, worin das Medikament in einer Menge von 1 bis 40 Gew.-% bezogen auf das Gewicht der druckempfindlichen Haftschicht zugegen ist.

**13.** Medizinische Zubereitung auf Acrylsäureestergel-Basis nach Anspruch 11, worin das Medikament eine Löslichkeit in der druckempfindlichen Haftschicht von wenigstens 4 Gew.-% aufweist.

**14.** Medizinische Zubereitung auf Acrylsäureestergel-Basis nach Anspruch 11, worin das Medikament eine Löslichkeit in dem organischen flüssigen Bestandteil von wenigstens 3 Gew.- % aufweist.

**15.** Medizinische Zubereitung auf Acrylsäureestergel-Basis nach Anspruch 11, worin die druckempfindliche Haftschicht eine Haftfestigkeit von 20 bis 180 g/12 mm Breite aufweist, gemessen mit einem Test zur Bestimmung der Abschalfestigkeit unter einem Winkel von 180° bei konstanter Geschwindigkeit, nachdem diese auf eine zweite druckempfindliche Haftschicht aufgebracht wurde.

**16.** Medizinische Zubereitung auf Acrylsäureestergel-Basis nach Anspruch 11, worin die druckempfindliche Haftschicht einen bei Scherungsbeanspruchung auftretenden Distanzwert der vollständigen Verschiebung des 0,5- bis 6-fachen der Dicke der druckempfindlichen Haftschicht aufweist, wenn eine Scherungsbeanspruchung auf die druckempfindliche Haftschicht aufgebracht wird.

**17.** Medizinische Zubereitung auf Acrylsäureestergel-Basis nach Anspruch 11, worin die druckempfindliche Haftschicht eine Grenzflächen-Haftfestigkeit zwischen der druckempfindlichen Haftschicht und den Hornschicht-Zellen aufweist, die wesentlich niedriger ist als die Bindungsfestigkeit zwischen den Hornschicht-Zellen.

**Revendications**

**1.** Gel à base d'ester acrylique destiné à être appliqué sur la peau, comprenant un support et une couche auto-adhésive chimiquement réticulée formée sur au moins une face dudit support, ladite couche auto-adhésive réticulée comprenant : un polymère à base d'ester acrylique comprenant un ester d'alkyle d'acide (méth)acrylique ; et un composant liquide organique pour plastifier ledit polymère et donner de la souplesse à ladite couche auto-adhésive, ledit composant liquide organique étant compatible avec ledit polymère et étant au moins un liquide organique choisi dans le groupe constitué par les solvants organiques, les tensioactifs, les plastifiants, les hydrocarbures et les esters d'acides gras, ladite couche auto-adhésive étant chimiquement réticulée de façon à introduire dans ladite couche auto-adhésive chimiquement réticulée un taux de gel situé entre 50 % et 80 % en masse par rapport audit polymère.

**2.** Gel à base d'ester acrylique destiné à être appliqué sur la peau selon la revendication 1, dans lequel la réticulation chimique est pratiquée en utilisant un agent de réticulation choisi dans le groupe constitué par les alcoolates contenant du titane, les alcoolates contenant de l'aluminium, les chélates contenant du titane, les chélates contenant de l'aluminium et les isocyanates trifonctionnels.

**3.** Gel à base d'ester acrylique destiné à être appliqué sur la peau selon la revendication 2, dans lequel l'agent de réticulation chimique est utilisé en une quantité allant de 0,05 à 3,0 parties en masse pour 100 parties en masse du polymère à base d'ester acrylique.

4. Gel à base d'ester acrylique destiné à être appliqué sur la peau selon la revendication 1, dans lequel ledit polymère à base d'ester acrylique est un copolymère obtenu en copolymérisant 60 à 98 % en masse dudit ester d'alkyle de l'acide méth(acrylique) dont la partie alkyle contient 4 à 15 atomes de carbone et 2 à 40 % en masse d'un monomère copolymérisable avec ledit ester d'alkyle de l'acide (méth)acrylique.

5. Gel à base d'ester acrylique destiné à être appliqué sur la peau selon la revendication 4, dans lequel ledit monomère copolymérisable est au moins un monomère choisi dans le groupe composé de monomères contenant un groupe carboxyle, des acrylates contenant un groupe hydroxyle, des acrylates contenant un groupe amide, des acrylates contenant un groupe aminoalkyle, des acrylates contenant un groupe alcoxy et des monomères vinyliques.

6. Gel à base d'ester acrylique destiné à être appliqué sur la peau selon la revendication 1, dans lequel la quantité dudit composant liquide organique contenu dans la couche auto-adhésive est située entre 25 et 200 parties en masse pour 100 parties en masse du polymère à base d'ester acrylique.

7. Gel à base d'ester acrylique destiné à être appliqué sur la peau selon la revendication 6, dans lequel la quantité dudit composant liquide organique contenu dans la couche auto-adhésive est située entre 40 et 160 parties en masse pour 100 parties en masse du polymère à base d'ester acrylique.

8. Gel à base d'ester acrylique destiné à être appliqué sur la peau selon la revendication 1, dans lequel la couche auto-adhésive a une adhérence, quand elle est liée à une seconde couche auto-adhésive de composition identique, située entre 20 et 180 g/12 mm de large, adhérence mesurée par le test de résistance au décollement à vitesse constante à 180°.

9. Gel à base d'ester acrylique selon la revendication 1, dans lequel ladite couche auto-adhésive a une distance finale de déplacement induite par une déformation par cisaillement, quand on impose à ladite couche autoadhésive une contrainte de cisaillement, située entre 0,5 et 6 fois l'épaisseur de ladite couche auto-adhésive.

10. Gel à base d'ester organique destiné à être appliqué sur la peau selon la revendication 1, dans lequel la couche auto-adhésive a une adhérence interfaciale entre ladite couche auto-adhésive et les cellules de la couche cornée substantiellement inférieure à la force liant entre elles lesdites cellules de la couche cornée.

11. Préparation médicale à base d'un gel contenant un ester acrylique pour l'absorption percutanée, comprenant :

un support, et une couche auto-adhésive chimiquement réticulée formée sur au moins une face dudit support, ladite couche auto-adhésive réticulée comprenant : un polymère à base d'ester acrylique comprenant un ester d'alkyle d'acide (méth)acrylique ; et un composant liquide organique pour plastifier ledit polymère et donner de la souplesse à ladite couche autoadhésive, ledit composant liquide organique étant compatible avec ledit polymère et étant au moins un liquide organique choisi dans le groupe constitué par les solvants organiques, les tensioactifs, les plastifiants, les hydrocarbures et les esters d'acides gras, ladite couche auto-adhésive étant chimiquement réticulée de façon à introduire dans ladite couche auto-adhésive chimiquement réticulée un taux de gel situé entre 50% et 80 % en masse par rapport audit polymère et
un médicament contenu dans la couche auto-adhésive.

12. Préparation médicale à base de gel contenant un ester acrylique selon la revendication 11, dans laquelle ledit médicament est présent en une quantité allant de 1 à 40 % en masse par rapport à la masse de la couche auto-adhésive.

13. Préparation médicale à base de gel contenant un ester acrylique selon la revendication 11, dans laquelle ledit médicament a une solubilité dans ladite couche auto-adhésive d'au moins 4 % en masse.

14. Préparation médicale à base de gel contenant un ester acrylique selon la revendication 11, dans laquelle ledit médicament a une solubilité dans ledit composant liquide organique d'au plus 3 % en masse.

15. Préparation médicale à base de gel contenant un ester acrylique selon la revendication 11, dans laquelle ladite couche auto-adhésive a une adhérence, quand elle est liée à une seconde couche auto-adhésive, située entre 20 et 180 g/12 mm de large, adhérence mesurée par le test de résistance au décollement à vitesse constante à 180°.

16. Préparation médicale à base de gel contenant un ester acrylique selon la revendication 11, dans laquelle ladite couche auto-adhésive a une distance finale de déplacement induite par une déformation par cisaillement, quand on impose à ladite couche auto-adhésive une contrainte de cisaillement, située entre 0,5 et 6 fois l'épaisseur de ladite couche auto-adhésive.

17. Préparation médicale à base de gel contenant un ester acrylique selon la revendication 11, dans laquelle ladite couche auto-adhésive a une adhérence interfaciale entre ladite couche auto-adhésive et les cellules de la couche cornée substantiellement inférieure à la force liant entre elles lesdites cellules de la couche cornée.